# EUROPEAN PATENT SPECIFICATION

(11) **EP 0 919 251 B1**
(45) Date of publication and mention of the grant of the patent: **17.01.2007**
(21) Application number: 98309678.5
(22) Date of filing: 25.11.1998
(51) Int. Cl.: A61M 5/145

(54) **Front-loading power injector and method of loading flanged syringe therein**
Spritzenpumpe mit Frontladung und Verfahren zum Laden mit einer mit Flanschen versehenen Spritze
Injecteur à chargement frontal et méthode pour le chargement d'une seringue pourvue d'un rebord

(30) Priority: 26.11.1997 US 979003
(43) Date of publication of application: 02.06.1999
(73) Proprietor: LIEBEL-FLARSHEIM COMPANY, Cincinnati, Ohio 45237 (US)
(72) Inventor: Ziemba, Robert J., Cincinnati, Ohio 45213 (US); Smith, Mitchell, Cincinnati, Ohio 45211 (US); Neer, Charles, Cincinnati, Ohio 45202 (US)
(74) Representative: Findlay, Alice Rosemary

(56) References cited:
- WO-A-97/07841
- WO-A-97/36635
- US-A- 4 342 312
- US-A- 4 465 473
- US-A- 5 279 569

## Description

This invention relates to power injectors, and particularly to injectors of the front loading type, and to the loading of syringes into power injectors, particularly syringes of the flanged or otherwise rear loadable type.

Power injectors are devices used to inject fluids at programmed or otherwise controlled rates or pressures into patients. Important uses include computed tomography (CT) and angiography, where a radiopaque contrast medium is injected into a patient's vascular system to enhance diagnostic images. With power injectors, a motor-driven ram advances the plunger of a syringe under the control, for example, of a microprocessor to provide control of injection parameters such as flow rate, flow volume and timing. Such injectors are often loaded with sterile empty syringes that are filled by drawing fluid from a supply into the syringe through the syringe nozzle by using the ram to draw the syringe plunger backward. In other situations, the injectors are loaded with prefilled syringes in which the fluid has been packaged in the syringe by media manufacturers.

Syringes of both the empty and the prefilled types are available in more than one design or style. The type of design or style of the empty syringes that are used by a practitioner are typically the choice, in part, of the practitioner and, in part, of the those selecting the injection equipment being used. With prefilled syringes, however, the choices of the practitioner are limited to the designs and styles provided by the prefilled syringe manufacturer, which may be limited due to the need of the manufacturer to submit the syringe and contents to various governmental approval processes. The time and costs involved in such processes as well as the costs of providing alternative syringe containers for each injection fluid product place practical and financial restraints on the prefilled syringe manufacturer who might be attempting to provide a variety of physical syringe configurations in its product line.

A variety of syringe designs have been developed and are in use for both prefilled and user-fillable syringes. Many such syringes are provided with a radially outwardly projecting flange at their rearward ends which serve to hold or support the syringe against axial motion when force is applied between the flange and plunger by the power ram of the injector. Many syringes with radial flanges on their rearward ends were first developed for use in breech loading or rear loading injectors in which the rear loadable syringe is positioned behind a holder of the injector and translated forwardly, nozzle first, through an opening in the holder, so that the flange or other outwardly extending structure at the syringe rear will seat forwardly against the back surface of the holder. Usually the holder is in the form of a faceplate or door on the injector housing and opens by moving away from the injector housing, either in hinged or turret fashion, for the loading or unloading of the syringe into or from the injector. One such rear loading injector is described and illustrated in U.S. Patent No. 4,695,271.

Injectors of this rear loading type were for many years a standard of the health care industry.

More recently, the assignee of the present invention has provided a front loading injector that receives front loadable syringes. A front loading injector is one in which a front loadable syringe is positioned in front of an opening in an injector holder and loaded into the holder by translating the syringe rearwardly, back end first, into the holder. These front loading injectors have a number of advantages that make them highly preferred. One such advantage is the ability to load the injector manually with a simple one handed motion, by merely rearwardly translating the syringe into an opening in the front of the injector, without opening a loading door, and then twisting or otherwise manipulating the syringe to lock it in place. Such a front loading injector is described and illustrated in U.S. Patent No. 5,279,569.

Notwithstanding the desirability of using front loadable syringes and front loading power injectors, it is often necessary to utilize prefilled or other syringes, which may be available only in a breech or rear loadable type. To accommodate practitioners in such situations, breech loading capability has been provided for front loading injectors of the type disclosed in U.S. Patent No. 5,279,569, referred to above, by interchanging the syringe holding head structure of the front loading injector with a breech loading syringe holder that will accept flanged rear loadable syringes. The use of interchangeable heads nonetheless has required the practitioner to resort to the rear loading techniques of the older systems which still have the disadvantages that provided the motivation for their replacement.

WI97/36635 discloses a syringe adapter which permits use of syringes of various types, sizes and configurations to be used with a front-loading injector, includes a removable injector face plate having a releasable mounting mechanism for attaching the adapter face plate to a front-load injector, a reducing sleeve having a recess for receiving the distal flange of syringe, locking pivot arms pivotly attached to the reducing sleeve to releasably engage syringe flange, and a piston extension provided for extending the injector drive piston for engagement with syringe plunger.

WO97/07841 discloses a pressure jacket system which is used to permit the front loading and removal of a syringe from an injector, and includes a jacket cylinder having an open front end for receiving a syringe and a rear end coupled to the injector front face, first and second tie rods having rear ends pivotally attached to the injector front face by pivots and bushings, and a front retaining place attached to the front ends of the first and second tie rods, such that the retaining plate and tie rods are pivotable between a closed position for holding the syringe within the jacket cylinder and an open position to allow the insertion and removal of the syringe from the jacket cylinder.

Accordingly, there remains a need to provide the advantages of a front loading injector and the capability of using syringes, particularly prefilled syringes, of the rear loadable type, particularly those having flanged or other outwardly extending structure on their rearward ends that function to align or lock the syringe on the injector.

An objective of the present invention is to provide a power injector having the capability of being front-loaded with syringes, and more particularly with rear loadable, side loadable, hand operable and other syringes, including prefilled syringes and syringes having locking and aligning structure such as an outwardly extending flange at the rearward end thereof. Another objective of the present invention is to provide a front loading injector capable of being front loaded with syringes having flanges at the rearward end thereof and to provide a method of front loading such syringes into a power injector.

A further objective of the present invention is to provide a power injector, such as a front loading injector, with a alternative structure, such as a substitute syringe holder or faceplate, that will adapt an injector to be front loaded with syringes of the rear loadable type, particularly syringes having flanges at their rearward ends.

The present invention provides, in one aspect, a front-loading injector for supporting a syringe having an outwardly extending support structure on a rearward end thereof and for driving a plunger in the syringe forwardly to eject fluid out of the syringe, the injector comprising an injector housing having a front with an opening therein, an injection ram disposed in the housing and extendible through the opening in the housing to drive a plunger of a syringe mounted to the injector, and a locking structure including elements that are moveable toward and away from an axis through the opening between a retracted position sufficiently spaced from the axis through the opening to permit passage of the outwardly extending support structure on the rearward end of the syringe when inserted rearwardly into or removed forwardly from the opening and a locking position in which the elements contact the syringe immediately forward of the support structure toward the rearward end thereof to thereby lock the syringe to the injector, characterised in that the locking structure is adjacent the opening, and in that the elements are capable of being moved from the retracted position toward the locking position by twisting a syringe when the syringe is disposed in the opening.

The present invention provides, in another aspect, a syringe holder for removable attachment to a power injector and for enabling the injector for the front loading and removal of a syringe having a longitudinal axis and a radially outwardly extending support structure on the rearward end thereof, the syringe holder comprising a holder housing having an opening therein and a connector to removably interconnect the housing with a power injector, and a locking structure on the housing including elements that are moveable radially toward and away from an axis through the opening between a retracted position sufficiently spaced from the axis to permit passage of an outwardly extending support structure of the rearward end of the syringe and a locking position in which the elements contact the syringe forward of the support structure toward the rearward end thereof to thereby lock the syringe to the holder, characterised in that the locking structure is adjacent the opening, and in that the elements are capable of being moved from the retracted position toward the locking position by twisting a syringe when the syringe is disposed in the opening

The present invention provides, in a further aspect,a method of front-loading into a power injector a syringe having a front end, an opposing rearward end, a longitudinal axis extending between the front and rearward ends, and a support structure that is disposed at the rearward end and that extends outwardly away from the longitudinal axis, the method comprising disposing at least the rearward end of the syringe into a syringe receiving opening defined in the power injector and about which a syringe locking structure is disposed, and twisting the syringe about the longitudinal axis thereof after at least the rearward end is in the syringe receiving opening of the power injector, characterised in that the twisting of the syringe causes the elements of the syringe locking structure to move toward an axis through the opening lock the syringe to the injector.

According to a preferred embodiment, a syringe holder is provided with a syringe receiving opening or cavity therein that is of a size and shape that will receive the rearward end of a breech loadable syringe when the syringe is translated axially rearwardly into the opening. The opening is provided with syringe gripping structure that moves from the periphery of the opening against the body of the syringe, to lock, align and orient the syringe in the opening.

In accordance with the preferred embodiment of the invention, an iris-like syringe holding mechanism is provided, such as, for example, a gripping mechanism including a plurality of jaws is arranged around the opening in a outwardly retracted position on the holder and are linked together. The jaws preferably function in an iris-like fashion, to reduce the size of the opening to that of the syringe body, thereby gripping the body immediately forward of the rearward end of the syringe. Structure on the rearward end of the syringe, such as a radially outwardly extending flange, for example of a type typical of a breech loadable prefilled syringe, is gripped in a slot between the jaws and a stop at the back of the opening, so that the syringe is locked in the opening, is aligned with its axis on the centerline of the opening and is oriented generally perpendicularly to the injector housing wall and parallel to and aligned with the injector ram.

In the preferred embodiment of the invention, the holder is removable, replaceable or exchangeable with holders of other configurations that are provided for supporting syringes of differing shapes or types, or for receiving syringes of the front loadable or rear loadable types. In an alternative embodiment of the invention, the holder with a syringe holding mechanism having iris like holding elements is provided as part of an injector.

With the present invention, breech loadable syringes, particularly prefilled syringes having various configurations of flanges, tabs or other outwardly extending structure at their rearward ends, are capable of being front loaded into and removed from the power injectors, and thereby have all of the advantages of front loadability, including those of high speed syringe replacement, the ability to load or remove a syringe with one hand, and the ability to remove a syringe while injection tubing remains connected to the syringe tip, and other advantages discussed in the incorporated patents cited above. The syringe holder of the present invention is capable of holding syringes with such radially or other outwardly extending structure on their rearward ends as well as syringes of a variety of other configurations, including syringes that lock to the injector at their rearward ends or elsewhere at other locations on the syringe.

The invention will now be further described by way of example with reference to the accompanying drawings in which:
**Fig. 1** is a perspective view of a front loading power injector according to principles of the present invention.
**Fig. 2** is a partial cross-sectional view along line **3-3** of **Fig. 1**, showing the injector with its syringe gripping structure in a retracted position.
**Fig. 3** is a view, similar to **Fig. 2**, with the gripping structure in a closed or locked position.
**Fig. 4** is a cross-sectional view along line **4-4** of **Fig. 3**.
**Fig. 5** is a front view of an alternative embodiment of the power injector of **Fig. 1**, showing the syringe gripping structure in a retracted position.
**Fig. 6** is a view, similar to **Fig. 5,** with the gripping structure in a closed or locked position.
**Fig. 7** is a cross-sectional view along line **7-7** of **Fig. 6**.

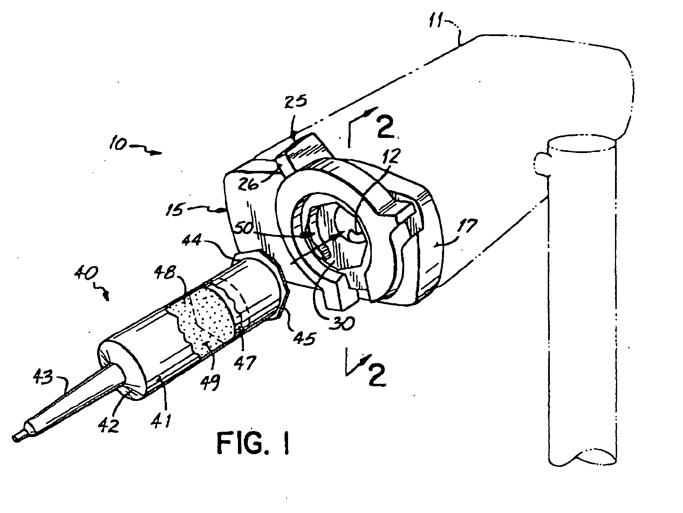

**Fig. 1** illustrates a power injector 10 of a type, for example, that is used for angiographic and CT injection. One such injector is described and illustrated in U.S. Patent No. 5,279,569, which is assigned to the assignee of the present invention, and is expressly incorporated herein by reference herein. The injector 10 includes an injector housing 11 that contains a power driven ram 12. The ram 12 is longitudinally extendable from the housing 11 in a direction normal to the front of the housing 11 by activation of a motor (not shown) contained within the housing 11. The forward end of the ram 12 is extendable from a retracted position in which its forward end is located inside the housing 11, as illustrated in Fig. 1, to an extended position in which its forward end has been advanced a considerable distance outside the front wall of the housing 11, as more fully explained in the incorporated U.S. Patent No. 5,279,569. The injector described in that cited patent has a door assembly that constitutes a syringe mounting head of a front loading type for receiving a front loadable syringe with the syringe being loaded therein by translating it rearwardly, back end first, into the front of the mounting head.

The embodiment of the invention illustrated in **Fig. 1** is, however, equipped with a faceplate in the form of a syringe mounting head 15 that is an alternative to, or a replacement for, or otherwise exchangeable with, the front loading head described in the patent which is configured to receive a syringe that is particularly designed for front loading, and which has a rearward end of the same outer diameter and cross-section as the syringe body. The mounting head 15 of the embodiment of **Fig. 1** is rather configured to receive a syringe 40 of a type that has been designed for breech loading into an injector of the rear loading type, where the syringe 40 is translated, front end first, through an opening from the rearward side of a loading door, which must be opened for the purpose of loading or unloading a syringe 40.

The syringe 40 typically has a cylindrical syringe body 41 with a frusto conical front end 42 having a conical injection nozzle 43 at the front thereof. The syringe body 41 has a rearward end 44 provided with a radially outwardly extending flange 45. The flange 45 is typically of uniform thickness and integrally formed with the syringe body 41 at its extreme rearward end. The flange 45 may be a ring shaped annular flange with a circular outer edge, but usually is provided with at least one flat edge or notch to angularly align the syringe or to prevent the syringe from rotating about its longitudinal axis. The flange 45 of the syringe 40 has a polygonal outer edge that will angularly orient, or prevent rotation of, the syringe 40 when mounted in an injector. As illustrated in **Fig. 1**, the syringe 40 is provided with a flange 45 having an outer edge of octagonal shape. Within the body 41 of the syringe 40 is a plunger 47 formed of a hard polymeric material which forms a slidable but sealable contact with the inside of the syringe body 41 and defines along with the body 41 a sealed cavity 48 within the body 41 which contains injection fluid 49, preferably the syringe 40 when supplied from the manufacturer, that is, a prefilled syringe.

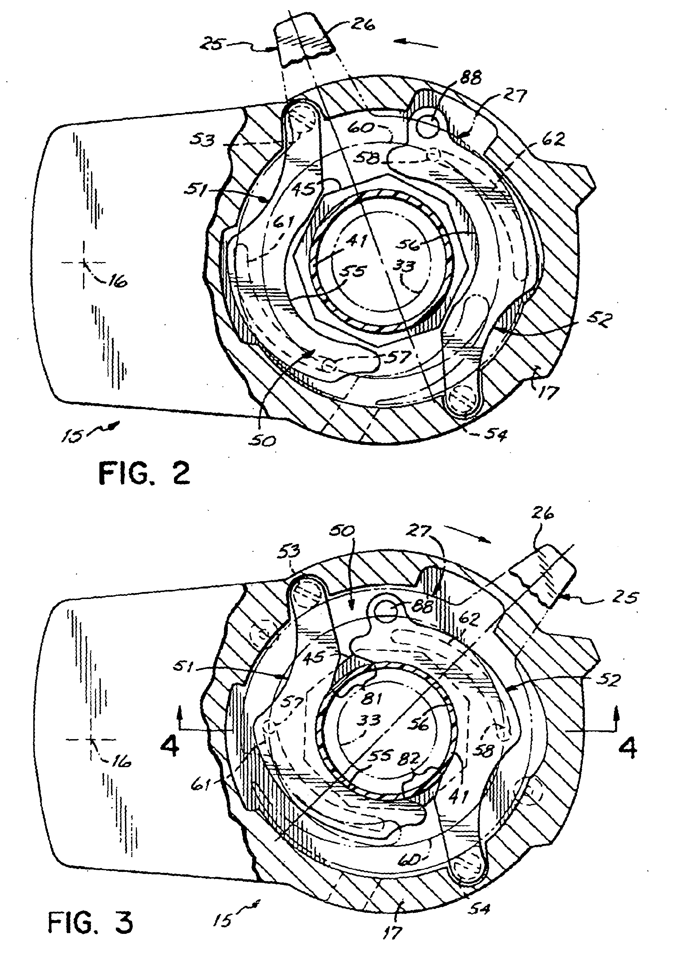

The head 15 preferably includes a casting 17 removably and pivotally connected to the front of the housing 11 to a pivot shaft 16 and with a locking mechanism 25, which is illustrated in part in Figs. 1-3, and as is more particularly described in the incorporated U.S. patent no, 5,279,569. The mechanism 25 functions to lock and unlock of the head 15 to and from the housing 11, to couple the ram 12 to plunger 47 within the body 41 of the syringe 40, and to lock the syringe 40 to the injector 10. The mechanism 25 is provided with an operating lever 26, illustrated in an unlocked or loading position in **Figs. 1** and **2.**, which is the position which allows the head 15 to be opened, removed and replaced and which allows syringes 40 to be loaded and unloaded from the mounting head 15. The lever or handle 26 is connected to a disc 27 rotatably mounted to the casting 17. The handle 26 is moveable to a locked position, illustrated in **Fig. 3**, which is the position at which the head 15 is locked to the injector 10, the ram 12 is coupled to the syringe ram and the syringe 40 is locked to the injector 10.

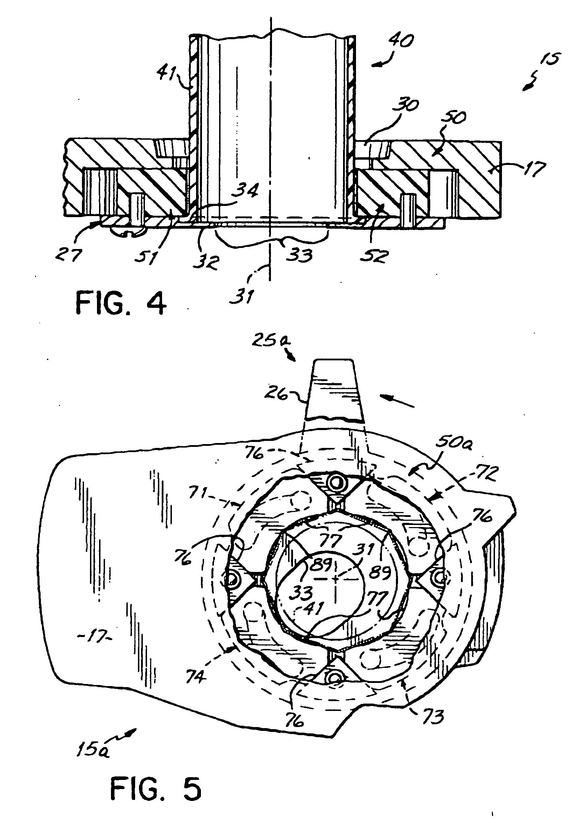

The casting 17 of the head 15 has an opening 30 therethrough, which is preferably of the same shape as the flange 45 of the syringe 40 and slightly larger in size to allow the flange 45 to be inserted into the opening 30. The opening 30 is preferably flared outwardly on the forward side thereof to guide the flange 45 of a syringe 40 into the opening 30 when the syringe 40 is being loaded. The opening 30 is centered on an axis 31 which is perpendicular to the front of the housing 11. The axis 31 is parallel to the path of the ram 12 and generally lies on the centerline of the ram 12 when the lever 26 is in its locked position. Behind the opening 30 is an annular stop 32 (**Fig. 4**), which lies in a plane that is oriented perpendicular to the axis 31. The stop 32 may be integrally formed of or otherwise fixed to the disc 27, which is centered to pivot on the axis 31. The stop 32 lies at the back of a recess 34 formed in the disc 27. The recess 34 has a depth approximately equal to the thickness of the flange 45. The recess 34 is preferably of the same shape as the flange 45 to hold the syringe 40 against rotation in the opening 30 once the syringe 40 is mounted in the opening 30. The stop 32 has an opening 33 therein to allow passage of the ram 12. When the flange 45 of a syringe 40 is inserted through the opening 30 and is seated against the stop 32, the axis of the syringe 40 coincides with the axis 31 of the opening 30, whereby the syringe 40 is perpendicular to the faceplate 15, and the syringe 40 is in its operating position on the injector 10.

According to one embodiment of the invention, the locking mechanism 25 includes a iris-like gripping mechanism 50 for holding the syringe 40 firmly in its operating position in the holder 15. The gripping mechanism 50 includes a plurality of gripping elements which preferably have gripping surfaces that encircle most of the circumference of the syringe body 31. In this embodiment, a pair of jaws 51,52, is provided, each jaw being pivotally connected to the housing 17 of the head 15 at respective pivot pins 53,54 which are fixed to the housing 17. The jaws 51,52 have inner concave gripping surfaces 55,56 having curvatures that correspond to the curvature of the outer surface of the body 41 of syringe 40. Preferably, the surfaces 55, 56 of the jaws 51,52 each encompass an arc of between 90° and 180°, so that when the jaws 51,52 are closed (**Fig. 3**) the surfaces 55,56 substantially surround the body 41 of the syringe 40 and securely hold the syringe in the operating position. The jaws 51,52 are spaced forward of the stop 32 so that, when the syringe 40 is in its operating position, the syringe flange 45 is held flat against the stop 32 by the jaws 51,52.

The jaws 51,52 are simultaneously moved between retracted positions (**Fig. 2**) and locked positions (**Fig. 3**) by a camming action of the mechanism 25, which is provided by a pair of cam follower pins 57,58 mounted on the ring 27 to move with and be actuated by the handle 26. A magnet 88 is provided on the jaw 52 which can be detected by a sensor (not shown) in the injector housing 11 in order to determine that the mechanism 25 is in its locked condition which enables, through controls (not shown) in the housing, the operation of the ram 12. The pins 57,58 scribe an arc on a circle 60, centered on the axis 31, when the handle 26 is operated. The pins 57,58 move in arcuate slots 61,62 in the jaws 51,52, respectively, so that, when the handle 26 is operated, the pins 57,58 move the slots 61,62 generally radially and thereby cam the jaws 51,52 to pivot on their respective pins 53,54 between their retracted positions, in which the surfaces 55,56 are displaced radially outward of the flange 45 to provide clearance for the flange 45 when the flange 45 moves between the opening 30 and the backplate 32 during loading and unloading, and their locked positions in which the jaws 51,52 seat snugly against the outside of the body 41 of the syringe 40 immediately forward of the flange 45. When so actuated, the jaws 51,52 move generally radially inwardly or outwardly along a path that may be, but is not necessarily normal to the outer surface 41 of the syringe 40.

According to another embodiment of the invention, an alternative head 15a is provided in which the syringe 40 is held in its operating position by an alternative gripping mechanism 50a having a set of four slidable jaws 71-74, as illustrated in **Figs 5-7**. Each of the jaws 71-74 has a rectangular cross-section when viewed from the axis 31, and each is trapped to slide radially in a respective one of four rectangular slots 75 formed between respective pairs of adjacent ones of a set of four wedges 76, which are fixed to the ring 27, and between the casting 17 and the rotatable ring 27. The jaws 71-74 have inner concave gripping surfaces 77 having curvatures that correspond to the curvature of the outer surface of the body 41 of syringe 40, so that when the jaws 71-74 are closed (**Fig. 3**) the surfaces 77 substantially surround the body 41 of the syringe 40 and securely hold the syringe in the operating position. The jaws 71-74 are spaced forward of the stop 32 so that, when the syringe 40 is in its operating position, the syringe flange 45 is held flat against the stop 32 by the jaws 71-74.

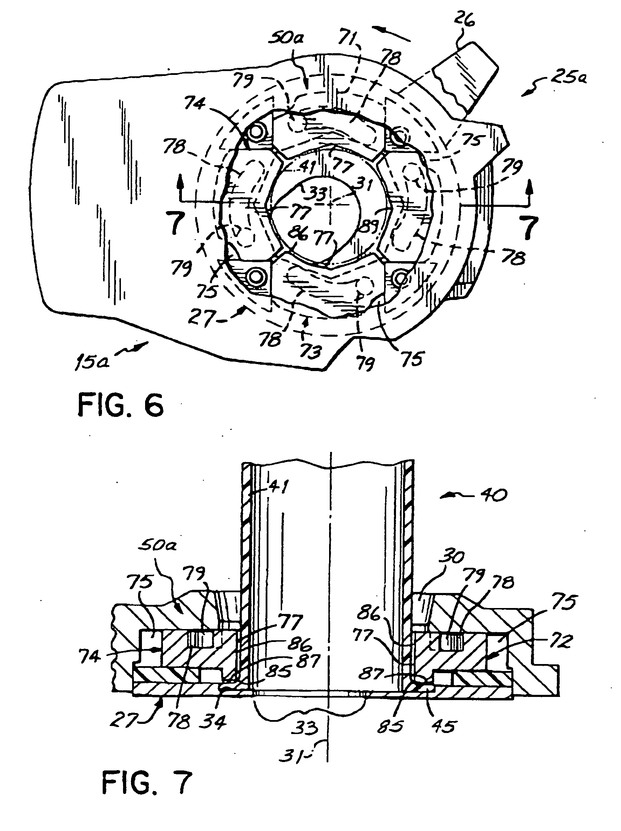

The jaws 71-74 rotate with the ring 27 and also simultaneously move between retracted positions (**Fig. 5**) and locked positions (**Fig. 6**) by the camming action of the mechanism 25a. The gripping surfaces 77 of the jaws 71-75 have V-shaped notches 89 therein which line up with the corners of the recess 34 in the ring 26. Each one of the jaws 71-74 is provided with a cam follower slot 78 in which is located a cam pin 79 which is fixed to the casting 17 and lies on a circle centered on the axis 31. When the handle 26 is operated to move the ring 27, the wedges 76 move with the ring 27 and rotate with them the jaws 71-74. The movement of the jaws 71-74 moves the slots 78 along the fixed pins 79, which causes the jaws 71-74 to move radially when the handle 26 is operated between their retracted positions, in which the gripping surfaces 77 are displaced radially outward of the flange 45 to provide clearance as the flange 45 during loading and unloading of the syringe 40, and their locked positions snugly against the outside of the body 41 of the syringe 40 immediately forward of the flange 45 to hold the syringe 40 in position for operation.

Preferably, as illustrated in **Fig. 3**, the gripping surfaces, such as surfaces 55,56 of jaws 51,52, span at least approximately 25% of the circumference of the syringe for many syringes. In the embodiment illustrated, the jaws 51,52 have spaces 81,82 between them that each span no more than approximately 10-20% of the circumference. With some syringes, it is preferred that the gripping surfaces span at least approximately 65% of the circumference and in addition or in the alternative, that spaces between contacting portions of the surfaces by not more than approximately 95% of the circumference of the syringe. For example, surfaces 77 of jaws 71-74 in **Fig. 6**, each have two arcs spaced by a V-shaped notch 89 of less than about 3 to 5% of the circumference. The spaces 83 between the jaws 71-74, when the jaws are in the locked position, also span less than approximately 3 to 5% of the circumference, leaving about 65% of the circumference cumulatively spanned by the surfaces 77.

While the jaws 51,52 and 71-74 are referred to herein as gripping jaws, these jaws may or may not actually squeeze radially inward on the syringe 40. What is preferred is that they contribute to the holding of the syringe in the opening 30, particularly resisting the force exerted by the ram 12 in moving the plunger 47. It is also important that, whatever holding action is performed by the gripping structure, the syringe 40 be held against the forces of operation without subjecting it to such forces and stresses that would cause any portion of the syringe 40 to fail. To this end, it is preferred that the jaws 51,52 or 71-74 engage the syringe 40 close to the body 41 of the syringe 40, at the intersection of the flange 45 with the syringe body 41, in a way that concentrates axial forces on the syringe 40 on the flange 45 as close to the body 41 as possible. This may be accomplished by configuring the rear edge of the inner surfaces 55, 56 or 77 of the jaws 51,52 or 71-74 with a smaller radius than the radius 85 between the flange 45 and the outside of the syringe body 41, as illustrated in **Fig. 6.** This leaves small spaces 86 between the jaws and the syringe body 41 and spaces 87 between the jaws and the syringe flange 45.

To load a syringe 40 into an injector 10, a faceplate 15 or 15a having a gripping mechanism 50 or 50a is mounted onto the injector housing 11, for example as a replacement to the standard faceplate with which the injector 10 is provided. If the syringe 40 has a plunger coupling that is not compatible with the coupling in the ram 12 of the injector 10, a ram coupling adapter may be attached to the end of the ram 12 to provide coupling between the ram 12 and the coupling element (not shown) on the rear side of the plunger 47.

With the lever 26 in its unlocked position, a syringe 40 is loaded into the opening 30 by translating the syringe 40 in a rearward axial direction, rear end first, until the flange 45 on the rearward end 44 of the syringe 40 passes through the opening 30 and rests in the recess 34 and against the stop plate 32 in the ring 27. The tapered front of the opening 30 in the casting 17 guides the flange 45 such that the shaped perimeter thereof lines up with the similarly shaped periphery of the recess 34. With the lever 26 so unlocked, the jaws 51,52 or 71-74 are in their retracted positions out of the path of the flange 45 as it is inserted into the opening.

When the rearward end 44 of the syringe 40 is completely seated in the opening, the lever 26 is moved to its locked position, which causes the jaws 51,52 or 71-74, to move to their closed positions against the outside of the body 41 of the syringe 40, gripping the body 41 and trapping the flange 45 between the jaws 51,52 or 71-74 and the ring 27. This locking motion securely holds the syringe in position in the holder 15 on the housing 11 of the injector 10, with its plunger 47 in line with the ram 12 and its centerline on the centerline of the holder 31 in alignment with the centerline of the path of the ram 12.

Removal of the syringe is accomplished by moving the lever 26 to its unlocked position to move the jaws 51,52 or 71-74 to their retracted positions. With the injector that is disclosed in U.S. Patent No. 5,279,569, this movement of the lever causes the locking mechanism 25,25a to slightly rotate the head 15 on the housing 11, which translates the ram 12 out of alignment with the centerline 31 of the opening, thereby shifting the syringe 40 sideways and uncoupling the ram 12 from the syringe plunger 47. The syringe 40 can thereby be manually removed from the injector 10 by translating it forwardly in the axial direction. If there is used injection tubing connected to the nozzle 43 after the use of the syringe 40, the syringe 40 can nonetheless be removed without disconnecting the tubing from the syringe nozzle 43.

Insertion of the syringe 40 into the injector 10 can also be carried out with a simple one handed motion, without separately operating the lever 26. This is accomplished by inserting the syringe in the opening 30 and translating it axially rearwardly until the flange 45 is seated against the stop plate **32** in the recess 34. Then by twisting the syringe 40 in the opening by less than 90°, preferably by about 45°, the ring 26 will turn, operating the locking mechanism 25, which moves the faceplate 15 and syringe plunger 47 into alignment with the ram 12 and operates the gripping mechanism 50,50a to move the jaws 51,52 or 71-74 to their locked position.

The invention provides the user of a power injector with the ability to front load an otherwise rear loadable syringe, particularly a rear loadable syringe having a flange at the rearward end thereof. This provides the user with the advantages of front loadability.

## Claims

1. A front-loading injector (10) for supporting a syringe (40) having an outwardly extending support structure (45) on a rearward end (44) thereof and for driving a plunger (47) in the syringe forwardly to eject fluid out of the syringe, the injector comprising an injector housing (11) having a front with an opening (30) therein, an injection ram (12) disposed in the housing (11) and extendible through the opening (30) in the housing to drive a plunger (47) of a syringe (40) mounted to the injector, and a locking structure (25, 25a) including elements (51, 52, 71-74) that are moveable toward and away from an axis (31) through the opening (30) between a retracted position sufficiently spaced from the axis (31) through the opening (30) to permit passage of the outwardly extending support structure (45) on the rearward end (44) of the syringe (40) when inserted rearwardly into or removed forwardly from the opening (30) and a locking position in which the elements (51, 52, 71-74) contact the syringe (40) immediately forward of the support structure (45) toward the rearward end (44) thereof to thereby lock the syringe (40) to the injector (10), **characterised in that** the locking structure (25, 25a) is adjacent the opening, and **in that** the elements (51, 52, 71-74) are capable of being moved from the retracted position toward the locking position by twisting a syringe (40) when the syringe (40) is disposed in the opening (30).

2. A syringe holder for removable attachment to a power injector (10) and for enabling the injector for the front loading and removal of a syringe (40) having a longitudinal axis and a radially outwardly extending support structure (45) on the rearward end (44) thereof, the syringe holder comprising a holder housing (15, 15a) having an opening (30) therein and a connector to removably interconnect the housing with a power injector, and a locking structure (25, 25a) on the housing including elements (51, 52, 71-74) that are moveable radially toward and away from an axis (31) through the opening (30) between a retracted position sufficiently spaced from the axis to permit passage of an outwardly extending support structure (45) of the rearward end (44) of the syringe (40) and a locking position in which the elements (51, 52, 71-74) contact the syringe (40) forward of the support structure (45) toward the rearward end (44) thereof to thereby lock the syringe (40) to the holder (10), **characterised in that** the locking structure (25, 25a) is adjacent the opening, and **in that** the elements (51, 52, 71-74) are capable of being moved from the retracted position toward the locking position by twisting a syringe (40) when the syringe (40) is disposed in the opening (30).

3. An injector or holder as claimed in Claim 1 or Claim 2 wherein the elements (51, 52, 71-74) when in the locking position, are in contact with a forward facing surface on the support structure (45) at the rearward end (44) of the syringe.

4. An injector or holder as claimed in any preceding claim wherein the elements each have a syringe engaging surface (55, 56, 77), each element being mounted to the injector (10) to move the syringe engaging surfaces between their retracted and their locking positions.

5. An injector or holder as claimed in any preceding claim wherein the elements have concave syringe engaging surfaces (55, 56, 77) conforming approximately to the syringe.

6. An injector or holder as claimed in any preceding claim wherein the elements have syringe engaging surfaces (55, 56, 77) thereon which, when in the locking position, contact a flange (45) of a syringe (40) inserted in the opening primarily at the intersection of the flange (45) with the body of the syringe.

7. An injector or holder as claimed in any preceding claim wherein the elements have syringe engaging surfaces (55, 56, 77) thereon cumulatively spanning at least approximately 25% of the circumference of the body of a syringe (40) inserted in the opening (30).

8. An injector or holder as claimed in any preceding claim wherein the elements have syringe engaging surfaces (55, 56, 77) thereon which, when in locking position around a syringe (40) that is held in the opening (30) have spaces (81, 82) therebetween that are each not more than approximately 5% of the circumference of the body of a syringe.

9. An injector or holder as claimed in any preceding claim wherein the locking structure includes two elements (51, 52) each having a syringe engaging surface (55, 56) which, when the elements are in the locking position, extend in an arc more than 90° around the syringe.

10. An injector or holder as claimed in any one of Claims 1 to 8 wherein the locking structure includes a plurality of more than two elements (71-74) each having a syringe engaging surface (77) such that, when the elements are in the locking position, the surfaces cumulatively extend in an arc of more than 180° around the syringe.

11. An injector or holder as claimed in any preceding claim wherein each of the elements has a slot (61, 62, 75) wherein, and the injector (10) or holder housing (15, 15a) includes a pin (57, 58, 79) to move in the slot of each of the elements (51, 52, 71-74), to move the elements between the retracted and locking positions.

12. An injector or holder as claimed in any preceding claim further comprising a camming mechanism (57, 58, 61, 62, 78, 79) to facilitate movement of the elements (51, 52, 71-74) between the locking and retracted positions.

13. A method of front-loading into a power injector (10) a syringe (40) having a front end, an opposing rearward end (44), a longitudinal axis extending between the front and rearward ends, and a support structure (45) that is disposed at the rearward end (44) and that extends outwardly away from the longitudinal axis, the method comprising disposing at least the rearward end (44) of the syringe (40) into a syringe receiving opening (30) defined in the power injector and about which a syringe locking structure (25, 25a) is disposed, and twisting the syringe (40) about the longitudinal axis thereof after at least the rearward end (44) is in the syringe receiving opening (30) of the power injector, **characterised in that** the twisting of the syringe (40) causes elements (51, 52, 71-74) of the syringe locking structure (25, 25a) to move toward an axis (31) through the opening (30) to lock the syringe (40) to the injector (10).

14. A method as claimed in Claim 13 wherein the twisting of the syringe (40) causes the elements (51, 52, 71-74) of the syringe locking structure (25, 25a) to move toward a center of the syringe receiving opening (30).

15. A method as claimed in Claim 13 or Claim 14 wherein the twisting of the syringe (40) causes the elements (51, 52, 71-74) of the syringe locking structure (25, 25a) to move toward the longitudinal axis of the syringe.

16. A method as claimed in any one of Claims 13 to 15 wherein the twisting of the syringe (40) causes the elements (51, 52, 71-74) of the syringe locking structure (25, 25a) to move in an iris-like fashion.

17. A method as claimed in any one of Claims 13 to 16 wherein the twisting of the syringe (40) causes camming action of the elements (51, 52, 71-74) of the syringe locking structure (25, 25a).

18. A method as claimed in any one of Claims 13 to 17 wherein the twisting of the syringe (40) includes twisting the syringe (40) less than 90 degrees.

## Patentansprüche

1. Frontlader-Injektor **(10)** zum Halten einer Spritze **(40),** die eine sich nach außen erstreckende Haltestruktur **(45)** an ihrem hinteren Ende **(44)** aufweist und zum Vorschieben eines Plungers **(47)** in der Spritze dient, um ein Fluid aus der Spritze auszustoßen, wobei der Injektor umfasst ein Injektorgehäuse **(11)** mit einer Vorderseite mit einer darin befindlichen Öffnung **(30),** einen Injektionskolben **(12),** der im Gehäuse **(11)** angeordnet und durch die Öffnung **(30)** im Gehäuse hindurch ausfahrbar ist, um einen auf den Injektor aufgesetzten Plunger **(47)** einer Spritze **(40)** vorzutreiben, und eine Arretierungskonstruktion **(25, 25a)** mit Elementen **(51, 52, 71 - 74),** die hin zu und weg von einer durch die Öffnung **(30)** verlaufenden Achse **(31)** beweglich sind zwischen einer zurückgezogenen Position, die von der durch die Öffnung **(30)** verlaufenden Achse **(31)** ausreichend weit entfernt ist, um den Durchgang der sich nach außen erstreckenden Haltestruktur **(45)** am hinteren Ende **(44)** der Spritze **(40)** zu gestatten, wenn sie rückwärts in die Öffnung **(30)** eingesetzt oder vorwärts aus dieser herausgenommen wird, und einer Arretierungsposition, in der die Elemente **(51, 52, 71 - 74)** die Spritze **(40)** unmittelbar vor der Haltestruktur **(45)** an deren hinterem Ende **(44)** berühren, um **dadurch** die Spritze **(40)** am Injektor **(10)** zu arretieren, **dadurch gekennzeichnet, dass** sich die Arretierungskonstruktion **(25, 25a)** angrenzend an die Öffnung befindet und dass die Elemente **(51, 52, 71 - 74)** durch ein Drehen der Spritze **(40)** von der zurückgezogenen Position in die Arretierungsposition bewegbar sind, wenn die Spritze **(40)** in der Öffnung (30) angeordnet ist.

2. Spritzenhalterung zur abnehmbaren Anbringung an einem Hochdruckinjektor **(10)** und zum Ermöglichen, dass an den Injektor eine Spritze **(40)** vorne angebracht und entfernt werden kann, die eine Längsachse und eine sich radial nach außen erstreckende Halterungsstruktur **(45)** an ihrem hinteren Ende **(44)** aufweist, wobei die Spritzenhalterung umfasst ein Halterungsgehäuse **(15, 15a)** mit einer Öffnung **(30)** darin und ein Verbindungsstück zur abnehmbaren Anbringung des Gehäuses an einen Hochdruckinjektor, und eine Arretierungskonstruktion **(25, 25a)** am Gehäuse mit Elementen **(51, 52, 71 - 74),** die hin zu und weg von einer durch die Öffnung **(30)** verlaufenden Achse **(31)** radial beweglich sind zwischen einer zurückgezogenen Position, die ausreichend weit von der Achse entfernt ist, um den Durchgang einer sich nach außen erstreckenden Halterungsstruktur **(45)** am hinteren Ende **(44)** der Spritze **(40)** zu gestatten, und einer Arretierungsposition, in der die Elemente **(51, 52, 71 - 74)** die Spritze **(40)** vor der Halterungsstruktur **(45)** an deren hinterem Ende **(44)** berühren, um **dadurch** die Spritze **(40)** in der Halterung **(10)** zu arretieren, **dadurch gekennzeichnet, dass** sich die Arretierungskonstruktion **(25, 25a)** angrenzend an die Öffnung befindet und dass die Elemente **(51, 52, 71 - 74)** durch ein Drehen der Spritze **(40)** von der zurückgezogenen Position in die Arretierungsposition bewegbar sind, wenn die Spritze **(40)** in der Öffnung (30) angeordnet ist.

3. Injektor oder Halterung nach Anspruch 1 oder Anspruch 2, wobei die Elemente **(51, 52, 71 - 74),** wenn sie sich in der Arretierungsposition befinden, mit einer nach vorne gerichteten Fläche an der Halterungsstruktur **(45)** am hinteren Ende **(44)** der Spritze in Kontakt sind.

4. Injektor oder Halterung nach einem der vorhergehenden Ansprüche, wobei die Elemente jeweils eine an die Spritze angreifende Fläche **(55, 56, 77)** aufweisen, wobei jedes Element an dem Injektor **(10)** so angebracht ist, dass es die an die Spritze angreifenden Flächen zwischen ihrer zurückgezogenen und ihrer Arretierungsposition bewegen kann.

5. Injektor oder Halterung nach einem der vorhergehenden Ansprüche, wobei die Elemente an die Spritze angreifende konkave Flächen **(55, 56, 77)** aufweisen, die annähernd der Form der Spritze entsprechen.

6. Injektor oder Halterung nach einem der vorhergehenden Ansprüche, wobei die Elemente an die Spritze angreifende Flächen **(55, 56, 77)** aufweisen, die, wenn sie sich in der Arretierungsposition befinden, einen Flansch **(45)** einer in die Öffnung eingesetzten Spritze **(40)** in erster Linie an der Schnittstelle zwischen dem Flansch **(45)** und dem Spritzenkörper berühren.

7. Injektor oder Halterung nach einem der vorhergehenden Ansprüche, wobei die Elemente an die Spritze angreifende Flächen **(55, 56, 77)** aufweisen, die zusammen mindestens 25 % des Umfanges des Körpers einer in die Öffnung **(30)** eingesetzten Spritze **(40)** umspannen.

8. Injektor oder Halterung nach einem der vorhergehenden Ansprüche, wobei die Elemente an die Spritze angreifende Flächen **(55, 56, 77)** aufweisen, die, wenn sie sich in der Arretierungsposition um eine in der Öffnung **(30)** gehaltenen Spritze **(40)** herum befinden, Zwischenräume **(81, 82)** zwischen einander aufweisen, die jeweils nicht mehr als annähernd 5 % des Umfanges des Körpers einer Spritze betragen.

9. Injektor oder Halterung nach einem der vorhergehenden Ansprüche, wobei die Arretierungskonstruktion zwei Elemente **(51, 52)** umfasst, die jeweils eine an die Spritze angreifende Fläche **(55, 56)** aufweisen, die sich, wenn sich die Elemente in der Arretierungsposition befinden, über einen Kreisbogen von mehr als 90° um die Spritze erstrecken.

10. Injektor oder Halterung nach einem der Ansprüche 1 bis 8, wobei die Arretierungskonstruktion eine größere Vielzahl von mehr als zwei Elementen **(71 - 74)** umfasst, die jeweils eine an die Spritze angreifende Fläche **(77)** aufweisen, so, dass sich die Flächen insgesamt über einen Bogen von mehr als 180° um die Spritze erstrecken, wenn sich die Elemente in der Arretierungsposition befinden.

11. Injektor oder Halterung nach einem der vorhergehenden Ansprüche, wobei jedes der Elemente einen Schlitz **(61, 62, 75)** aufweist und der Injektor **(10)** oder das Gehäuse der Halterung **(15, 15a)** einen Stift **(57, 58, 79)** enthält, der sich in dem Schlitz jedes der Elemente **(51, 52, 71 - 74)** bewegen kann, um die Elemente zwischen der zurückgezogenen und der arretierenden Position zu bewegen.

12. Injektor oder Halterung nach einem der vorhergehenden Ansprüche, ferner mit einem Kurvengetriebe **(57, 58, 61, 62, 78, 79)** zur Erleichterung der Bewegung der Elemente **(51, 52, 71 - 74)** zwischen der zurückgezogenen und der arretierenden Position.

13. Verfahren zum Frontladen einer Spritze **(40)** in einen Hochdruckinjektor **(10),** die ein vorderes Ende und ein gegenüberliegendes hinteres Ende **(44),** eine Längsachse, die sich zwischen vorderem und hinterem Ende erstreckt, und eine Halterungsstruktur **(45)** aufweist, die am hinteren Ende **(44)** angebracht ist und sich von der Längsachse aus nach außen erstreckt, wobei das Verfahren umfasst ein Anordnen von mindestens dem hinteren Ende **(44)** der Spritze **(40)** in eine die Spritze aufnehmende Öffnung **(30),** die sich im Hochdruckinjektor befindet und um die herum innen eine Spritzenarretierungskonstruktion **(25, 25a)** angeordnet ist, und das Drehen der Spritze **(40)** um ihre Längsachse, nachdem sich mindestens das hintere Ende **(44)** in der die Spritze aufnehmenden Öffnung **(30)** des Hochdruckinjektors befindet, **dadurch gekennzeichnet, dass** das Drehen der Spritze **(40)** die Elemente **(51, 52, 71 - 74)** der Spritzenarretierungskonstruktion **(25, 25a)** dazu veranlasst, sich durch die Öffnung **(30)** hindurch hin zu einer Achse **(31)** zu bewegen, um die Spritze **(40)** am Injektor **(10)** zu arretieren.

14. Verfahren nach Anspruch 13, wobei das Drehen der Spritze **(40)** die Elemente **(51, 52, 71 - 74)** der Spritzenarretierungskonstruktion **(25, 25a)** dazu veranlasst, sich hin zu einem Zentrum der die Spritze aufnehmenden Öffnung **(30)** zu bewegen.

15. Verfahren nach Anspruch 13 oder Anspruch 14, wobei das Drehen der Spritze **(40)** die Elemente **(51, 52,71 - 74)** der Spritzenarretierungskonstruktion **(25, 25a)** dazu veranlasst, sich hin zur Längsachse der Spritze zu bewegen.

16. Verfahren nach einem der Ansprüche 13 bis 15, wobei das Drehen der Spritze **(40)** die Elemente **(51, 52, 71 - 74)** der Spritzenarretierungskonstruktion **(25, 25a)** dazu veranlasst, sich in einer irisartigen Weise zu bewegen.

17. Verfahren nach einem der Ansprüche 13 bis 16, wobei das Drehen der Spritze **(40)** eine kurvengesteuerte Aktion der Elemente **(51, 52, 71 - 74)** der Spritzenarretierungskonstruktion **(25, 25a)** veranlasst.

18. Verfahren nach einem der Ansprüche 13 bis 17, wobei das Drehen der Spritze **(40)** ein Drehen der Spritze **(40)** um weniger als 90 Grad umfasst.

## Revendications

1. Injecteur à chargement par l'avant (10) destiné à supporter une seringue (40) ayant une structure de support s'étendant vers l'extérieur (45) sur son extrémité arrière (44) et à entraîner un piston plongeur (47) dans la seringue vers l'avant pour éjecter du fluide à l'extérieur de la seringue, l'injecteur comportant un boîtier d'injecteur (11) ayant un avant muni d'une ouverture (30) dans celui-ci, un piston d'injection (12) disposé dans le boîtier (11) et pouvant s'étendre à travers l'ouverture (30) du boîtier pour entraîner le piston plongeur (47) d'une seringue (40) montée sur l'injecteur, et une structure de verrouillage (25, 25a) incluant des éléments (51, 52, 71 à 74) qui sont mobiles vers un axe (31), et en s'éloignant de celui-ci, à travers l'ouverture (30), entre une position rétractée suffisamment espacée de l'axe (31) à travers l'ouverture (30) pour permettre le passage de la structure de support s'étendant vers l'extérieur (45) sur l'extrémité arrière (44) de la seringue (40) lorsqu'elle est insérée vers l'arrière dans l'ouverture (30) ou enlevée vers l'avant à partir de celle-ci et une position de verrouillage dans laquelle les éléments (51, 52, 71 à 74) viennent en contact avec la seringue (40) immédiatement à l'avant de la structure de support (45), dans la direction de l'extrémité arrière (44) de celle-ci, pour ainsi verrouiller la seringue (40) sur l'injecteur (10), **caractérisé en ce que** la structure de verrouillage (25, 25a) est adjacente à l'ouverture, et **en ce que** les éléments (51, 52, 71 à 74) peuvent être déplacés depuis la position rétractée vers la position de verrouillage en tournant une seringue (40) lorsque la seringue (40) est disposée dans l'ouverture (30).

2. Support de seringue pour fixation amovible sur un injecteur électrique (10) et pour permettre à l'injecteur un chargement et un enlèvement par l'avant d'une seringue (40) ayant un axe longitudinal et une structure de support s'étendant radialement vers l'extérieur (45) sur son extrémité arrière (44), le support de seringue comportant un boîtier de support (15, 15a) ayant une ouverture (30) dans celui-ci et un connecteur pour relier mutuellement de manière amovible le boîtier à un injecteur électrique, et une structure de verrouillage (25, 25a) sur le boîtier incluant des éléments (51, 52, 71 à 74) qui sont mobiles radialement en direction d'un axe (31) et en s'éloignant de celui-ci à travers l'ouverture (30) entre une position rétractée suffisamment espacée de l'axe pour permettre le passage de la structure de support s'étendant vers l'extérieur (45) de l'extrémité arrière (44) de la seringue (40) et une position de verrouillage dans laquelle les éléments (51, 52, 71 à 74) viennent en contact avec la seringue (40) à l'avant de la structure de support (45) dans la direction de l'extrémité arrière (44) de celle-ci, pour ainsi verrouiller la seringue (40) sur le support (10), **caractérisé en ce que** la structure de verrouillage (25, 25a) est adjacente à l'ouverture, et **en ce que** les éléments (51, 52, 71 à 74) peuvent être déplacés depuis la position rétractée vers la position de verrouillage en tournant une seringue (40) lorsque la seringue (40) est disposée dans l'ouverture (30).

3. Injecteur ou support selon la revendication 1 ou 2, dans lequel les éléments (51, 52, 71 à 74) lorsqu'ils sont dans la position de verrouillage, sont en contact avec une surface dirigée vers l'avant de la structure de support (45) au niveau de l'extrémité arrière (44) de la seringue.

4. Injecteur ou support selon l'une quelconque des revendications précédentes, dans lequel les éléments ont chacun une surface de contact avec la seringue (55, 56, 77), chaque élément étant monté sur l'injecteur (10) pour déplacer les surfaces de contact avec la seringue entre leurs positions rétractées et leurs positions de verrouillage.

5. Injecteur ou support selon l'une quelconque des revendications précédentes, dans lequel les éléments ont des surfaces de contact avec la seringue concaves (55, 56, 77) se conformant approximativement à la seringue.

6. Injecteur ou support selon l'une quelconque des revendications précédentes, dans lequel les éléments ont des surfaces de contact avec la seringue (55, 56, 77) sur lesquelles, lorsqu'elles sont dans la position de verrouillage, est en contact un rebord (45) d'une seringue (40) insérée dans l'ouverture, principalement au niveau de l'intersection du rebord (45) avec le corps de la seringue.

7. Injecteur ou support selon l'une quelconque des revendications précédentes, dans lequel les éléments ont des surfaces de contact avec la seringue (55, 56, 77) sur ceux-ci, s'étendant de manière cumulée sur au moins approximativement 25 % de la circonférence du corps d'une seringue (40) insérée dans l'ouverture (30).

8. Injecteur ou support selon l'une quelconque des revendications précédentes, dans lequel les éléments ont des surfaces de contact avec la seringue (55, 56, 77) qui, lorsqu'elles sont dans une position de verrouillage autour d'une seringue (40) qui est maintenue dans l'ouverture (30), ont des espaces (81, 82) entre elles qui ne sont pas, chacun, plus grands qu'approximativement 5 % de la circonférence du corps d'une seringue.

9. Injecteur ou support selon l'une quelconque des revendications précédentes, dans lequel la structure de verrouillage comporte deux éléments (51, 52) ayant chacun une surface de contact avec la seringue (55, 56) qui, lorsque les éléments sont dans la position de verrouillage, s'étend sur un arc supérieur à 90° autour de la seringue.

10. Injecteur ou support selon l'une quelconque des revendications 1 à 8, dans lequel la structure de verrouillage comporte une pluralité constituée de plus de deux éléments (71 à 74) ayant chacun une surface de contact avec la seringue (77) de telle sorte que, lorsque les éléments sont dans la position de verrouillage, les surfaces s'étendent de manière cumulée sur un arc supérieur à 180° autour de la seringue.

11. Injecteur ou support selon l'une quelconque des revendications précédentes, dans lequel chacun des éléments comporte une fente (61, 62, 75) dans celui-ci, et l'injecteur (10) ou le boîtier de support (15, 15a) comporte un ergot (57, 58, 79) pour se déplacer dans la fente de chacun des éléments (51, 52, 71 à 74) pour déplacer les éléments entre les positions rétractées et de verrouillage.

12. Injecteur ou support selon l'une quelconque des revendications précédentes, comprenant en outre un mécanisme à effet de came (57, 58, 61, 62, 78, 79) pour faciliter un mouvement des éléments (51, 52, 71 à 74) entre les positions de verrouillage et rétractées.

13. Procédé de chargement par l'avant dans un injecteur électrique (10) d'une seringue (40) ayant une extrémité avant, et une extrémité arrière opposée (44), un axe longitudinal s'étendant entre les extrémités avant et arrière, et une structure de support (45) qui est disposée au niveau de l'extrémité arrière (44) et qui s'étend vers l'extérieur en s'éloignant de l'axe longitudinal, le procédé consistant à disposer au moins l'extrémité arrière (44) de la seringue (40) dans une ouverture de réception de seringue (30) définie dans l'injecteur électrique et autour de laquelle une structure de verrouillage de seringue (25, 25a) est disposée, et tourner la seringue (40) autour de son axe longitudinal après qu'au moins l'extrémité arrière (44) soit dans l'ouverture de réception de seringue (30) de l'injecteur électrique, **caractérisé en ce que** la rotation de la seringue (40) amène les éléments (51, 52, 71 à 74) de la structure de verrouillage de seringue (25, 25a) à se déplacer en direction d'un axe (31) à travers l'ouverture (30) pour verrouiller la seringue (40) sur l'injecteur (10).

14. Procédé selon la revendication 13, dans lequel la rotation de la seringue (40) amène les éléments (51, 52, 71 à 74) de la structure de verrouillage de seringue (25, 25a) à se déplacer vers le centre de l'ouverture de réception de seringue (30).

15. Procédé selon la revendication 13 ou 14, dans lequel la rotation de la seringue (40) amène les éléments (51, 52, 71 à 74) de la structure de verrouillage de seringue (25, 25a) à se déplacer vers l'axe longitudinal de la seringue.

16. Procédé selon l'une quelconque des revendications 13 à 15, dans lequel la rotation de la seringue (40) amène les éléments (51, 52, 71 à 74) de la structure de verrouillage de seringue (25, 25a) à se déplacer à la manière d'un diaphragme.

17. Procédé selon l'une quelconque des revendications 13 à 16, dans lequel la rotation de la seringue (40) amène une action d'effet de came des éléments (51, 52, 71 à 74) de la structure de verrouillage de seringue (25, 25a).

18. Procédé selon l'une quelconque des revendications 13 à 17, dans lequel la rotation de la seringue (40) inclut une mise en rotation de la seringue (40) plus petite que 90 degrés.
